# FASCICULE DE BREVET EUROPEEN

(11) **EP 2 241 553 B1**
(45) Date de publication et mention de la délivrance du brevet: **20.07.2011**
(21) Numéro de dépôt: 10290165.9
(22) Date de dépôt: 30.03.2010
(51) Int. Cl.: C07D 223/16

(54) **Procédé de synthèse de l'ivabradine et de ses sels d'addition à un acide pharmaceutiquement acceptable**
Verfahren zur Herstellung von Ivabradine und deren pharmazeutisch verträglichen Säureadditionssalzen
Process for the synthesis of ivabradine and its pharmaceutically acceptable acid addition salts

(30) Priorité: 31.03.2009 FR 0901556
(43) Date de publication de la demande: 20.10.2010
(73) Titulaire: Les Laboratoires Servier, 92284 Suresnes Cedex (FR)
(72) Inventeur: Peglion, Jean-Louis, 78110 Le Vésinet (FR); Dessinges, Aimée, 92500 Rueil-Malmaison (FR); Serkiz, Bernard, 77170 Servon Brie Comte Robert (FR)

(56) Documents cités:
- EP-A- 0 534 859
- EP-A- 1 598 333
- WO-A-2005/110993
- ROUSSELET ET AL: "Copper(I)-induced addition of amines to unactivated nitriles: The first general one-step synthesis of alkyl amidines" TETRAHEDRON LETTERS, ELSEVIER, AMSTERDAM, vol. 34, no. 40, 1 octobre 1993 (1993-10-01), pages 6395-6398, XP022406486 ISSN: 0040-4039
- BOMHARD A ET AL: "SPECIFIC BRADYCARDIC AGENTS. 2. HETEROAROMATIC MODIFICATIONS IN THE SIDE CHAIN OF SPECIFIC BRADYCARDIC BENZAZEPINONES: CHEMISTRY, PHARMACOLOGY, AND STRUCTURE-ACTIVITY RELATIONSHIPS" JOURNAL OF MEDICINAL CHEMISTRY, AMERICAN CHEMICAL SOCIETY, WASHINGTON, US, vol. 34, 1 janvier 1991 (1991-01-01), pages 942-947, XP002277379 ISSN: 0022-2623

## Description

La présente invention concerne un procédé de synthèse de l'ivabradine de formule (I) : 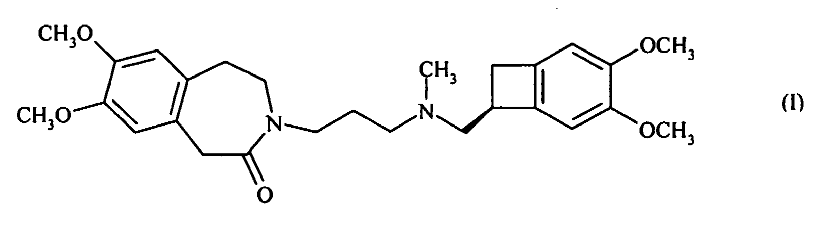 ou 3-{3-[{[(7*S*)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0 534 859.

Ce brevet décrit la synthèse du chlorhydrate de l'ivabradine à partir du composé de formule (II) : 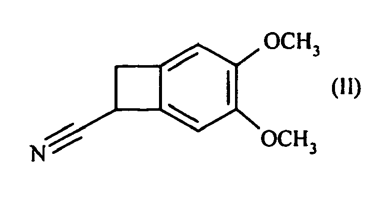 qui est soumis à une réaction de réduction pour conduire au composé de formule (III) : 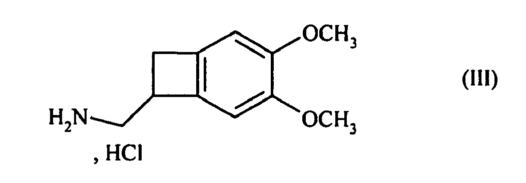 qui est transformé en composé de formule (IV) : 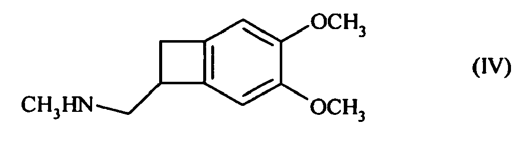 qui est dédoublé pour conduire au composé de formule (V) : 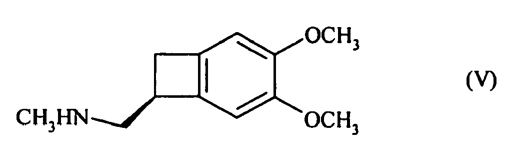 qui est mis en réaction avec le composé de formule (VI) : 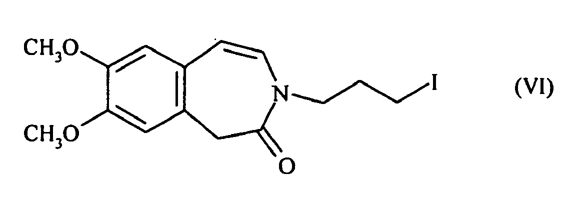 pour conduire au composé de formule (VII) : 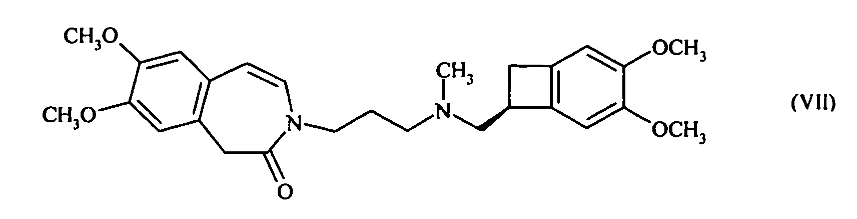 dont l'hydrogénation catalytique conduit à l'ivabradine, qui est alors transformée en son chlorhydrate.

L'inconvénient de cette voie de synthèse est de ne conduire à l'ivabradine qu'avec un rendement de l'ordre de 0,5%.

Compte-tenu de l'intérêt pharmaceutique de ce composé, il était important de pouvoir y accéder avec un procédé de synthèse performant, conduisant à l'ivabradine avec un bon rendement.

La présente invention concerne un procédé de synthèse du composé de formule (VIII), sous forme racémique ou optiquement active : 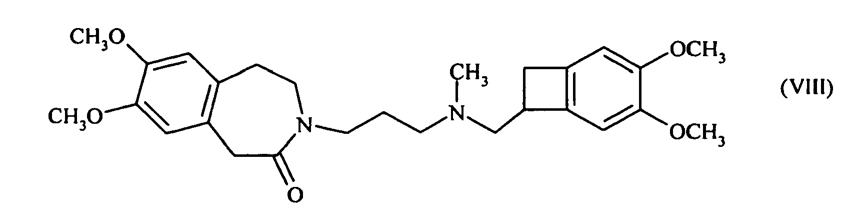 caractérisé en ce que le composé de formule (II), sous forme racémique ou optiquement active : 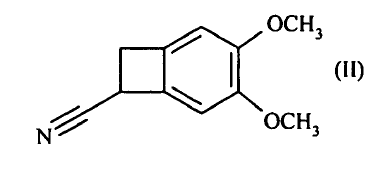 est mis en réaction avec le composé de formule (IX) : 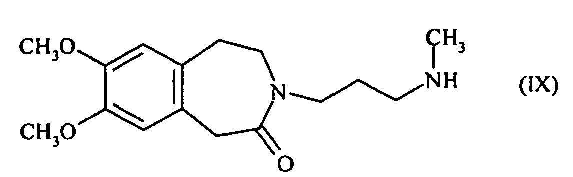 en présence d'un sel de métal de transition ou de lanthanide,
dans un solvant,
pour conduire au composé de formule (X), sous forme racémique ou optiquement active : 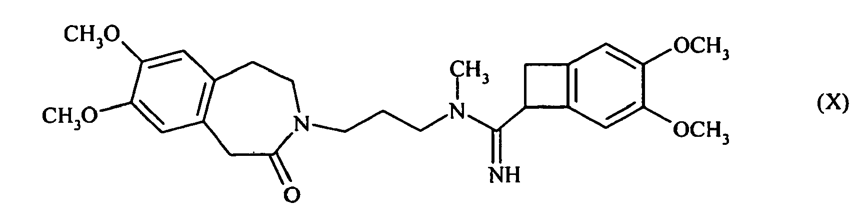 qui est transformé en composé de formule (VIII) par action d'un agent donneur d'hydrure.

Dans un mode de réalisation préféré de l'invention, le composé de formule (II) est sous forme optiquement active, et plus particulièrement de configuration (*S*). Le produit de la réaction du composé de formule (X) avec l'agent donneur d'hydrure est alors l'ivabradine de formule (I), cas particulier des composés de formule (VIII) : 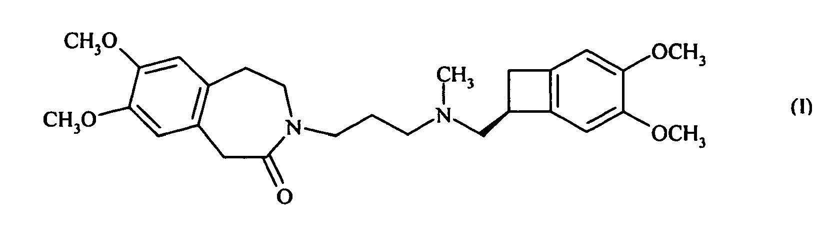 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Dans un autre mode de réalisation préféré de l'invention, le composé de formule (II) est sous forme racémique. La réaction du composé de formule (X) avec un agent donneur d'hydrure est alors suivie d'une étape de résolution optique du composé de formule (VIII) obtenu sous forme racémique, pour conduire à l'ivabradine de formule (I) : 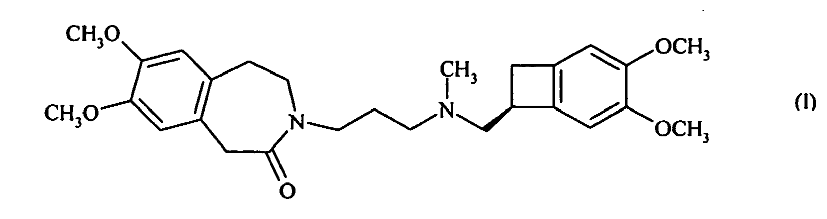 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Parmi les sels de métaux de transition ou de lanthanides pouvant être utilisés pour effectuer la réaction entre le composé de formule (II) et le composé de formule (IX), on peut citer à titre non limitatif le chlorure de cuivre(I), le bromure de cuivre(I), l'iodure de cuivre(I), le trifluorométhanesulfonate d'yttrium(III), le trifluorométhanesulfonate de lanthane(III), le trifluorométhanesulfonate de praséodyme(III), le trifluorométhanesulfonate de néodyme(III), le trifluorométhanesulfonate de samarium(III), le trifluorométhanesulfonate d'europium(III), le trifluorométhanesulfonate de gadolinium(III), le trifluorométhanesulfonate de terbium(III), le trifluorométhanesulfonate de dysprosium(III), le trifluorométhanesulfonate d'holmium(III), le trifluorométhanesulfonate d'erbium(III) et le trifluorométhanesulfonate de lutécium(III).

Le sel de métal de transition préférentiellement utilisé pour effectuer la réaction entre le composé de formule (II) et le composé de formule (IX) est le chlorure de cuivre(I).

Parmi les solvants pouvant être utilisés pour effectuer la réaction entre le composé de formule (II) et le composé de formule (IX), on peut citer à titre non limitatif :
- les solvants alcooliques, notamment le méthanol, l'éthanol, et l'isopropanol ;
- le diméthylsulfoxyde (DMSO) ;
- le *N,N*-diméthylformamide (DMF) ;
- la *N*-méthylpyrrolidone (NMP).

Le solvant préférentiellement utilisé pour effectuer la réaction entre le composé de formule (II) et le composé de formule (IX) est le méthanol.

Parmi les agents donneurs d'hydrure pouvant être utilisés pour effectuer la transformation du composé de formule (X) en composé de formule (VIII), on peut citer à titre non limitatif le tétraborohydrure de sodium, le cyanoborohydrure de sodium, ainsi que les complexes borane-morpholine et borane-diméthylamine.

L'agent donneur d'hydrure préférentiellement utilisé pour effectuer la transformation du composé de formule (X) en composé de formule (VIII) est le tétraborohydrure de sodium.

Parmi les solvants pouvant être utilisés pour effectuer la transformation du composé de formule (X) en composé de formule (VIII), on peut citer à titre non limitatif :
- les solvants alcooliques, notamment le méthanol, l'éthanol et l'isopropanol ;
- le *N,N*-diméthylformamide (DMF) ;
- la *N*-méthylpyrrolidone (NMP).

Les composés de formule (X), sous forme racémique ou optiquement active, sont des produits nouveaux, utiles comme intermédiaires de synthèse dans l'industrie chimique ou pharmaceutique, notamment dans la synthèse de ivabradine, de ses sels d'addition à un acide pharmaceutiquement acceptable et de leurs hydrates, et font à ce titre partie intégrante de la présente invention.

Les exemples ci-dessous illustrent l'invention.

### Liste des abréviations utilisées :

### IR : infrarouge

### EXEMPLE 1 : 3-{3-[[(3,4-diméthoxybicyclo[4.2.0]octa-1,3,5-trién-7-yl)méthyl](méthyl)amino]propyl}-7,8-diméthoxy-1,3,4,5-tétrahydro-2H-3-benzazépin-2-one

### Stade 1 : N-[3-(7,8-diméthoxy-2-oxo-1,2,4,5-tétrahydro-3H-3-benzazépin-3-yl)propyl]-3,4-diméthoxy-N-méthylbicyclo[4.2.0]octa-1,3,5-triène-7-carboximidamide

Sous azote, on solubilise 2g (10,6 mmoles) de 3,4-diméthoxybicyclo[4.2.0]octa-1,3,5-triène-7-carbonitrile dans 10 mL de méthanol. On ajoute à cette solution 1,3g (12,7 mmoles, 1,2 équivalent) de chlorure de cuivre(I) (pureté 99%). Puis on additionne goutte à goutte 4,6 g (15,9 mmoles, 1,5 équivalent) de 7,8-diméthoxy 3-[3-(méthylamino)propyl]-1,3,4,5-tétrahydro-2*H*-3-benzazépin-2-one en solution dans 20 mL de méthanol. On chauffe à reflux pendant 24 heures. On refroidit à 0°C, on ajoute de l'acide chlorhydrique concentré jusqu'à pH 2 et on agite 10 minutes pour décomplexer les sels de cuivre. On amène ensuite le pH à 8 en ajoutant une solution aqueuse de soude à 20%. La phase aqueuse est extraite au dichlorométhane, séchée sur MgSO₄, filtrée puis évaporée à sec. On obtient 6,4g d'une huile marron contenant 35% de produit attendu en mélange avec 21% d'amine de départ. Cette huile est engagée sans purification dans l'étape suivante.

### Stade 2 : 3-{3-[[(3,4-diméthoxybicyclo[4.2.0]octa-1,3,5-trién-7-yl)méthyl](méthyl)amino] propyl}-7,8-diméthoxy-1,3,4,5-tétrahydro-2H-3-benzazépin-2-one

On solubilise dans 65 mL de méthanol 6,4 g de produit obtenu au stade 1. Puis on ajoute, à 25°C, 481 mg (12,7 mmoles, 1,2 équivalent) de tétraborohydrure de sodium. On laisse agiter une nuit à température ambiante. On ajoute 26 mL de solution aqueuse de soude à 20% et 100 mL de dichlorométhane puis on agite vigoureusement 15 minutes. La phase organique est lavée à l'eau, séchée sur MgSO₄, filtrée puis évaporée à sec. On obtient 6,6 g d'une huile qui est purifiée par flash-chromatographie sur 300g de silice (éluant = dichlorométhane/éthanol/NH₄OH : 90/10/1). On obtient 1,6 g de produit du titre sous forme d'une huile qui cristallise à température ambiante.
Rendement = 33% (sur 2 étapes)
IR (pur): ν = 1633, 831, 672 cm⁻¹.

### EXEMPLE 2 : Chlorhydrate du 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

2,1 g du composé racémique obtenu à l'exemple précédent sont séparés sur une colonne de 60 cm x 60 mm packée avec 2,1 kg de phase Chiralpak ® AD (granulométrie 20 µm). L'éluant utilisé est un mélange éthanol / acétonitrile / diéthylamine (10 / 90 / 0,1 en volume) à un débit de 50 mL / min. Le détecteur ultra-violet associé est utilisé à un longueur d'onde de 280 nm.
On obtient 0,95g de l'énantiomère de configuration (*R*) sous forme de meringue blanche puis 0,95g de l'énantiomère de configuration (*S*) également sous forme de meringue blanche.

Le chlorhydrate de l'énantiomère de configuration (*S*) est ensuite obtenu en suivant le mode opératoire décrit dans le brevet EP 0 534 859 (Exemple 2, stade E).

## Revendications

1. Procédé de synthèse du composé de formule (VIII), sous forme racémique ou optiquement active : 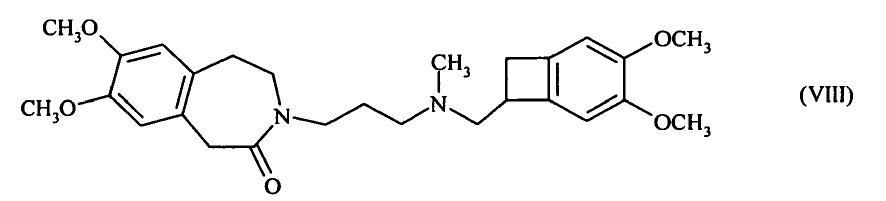 **caractérisé en ce que** le composé de formule (II), sous forme racémique ou optiquement active : 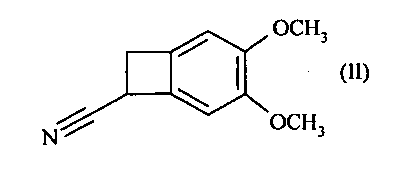 est mis en réaction avec le composé de formule (IX) : 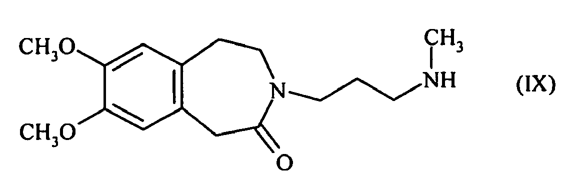 en présence d'un sel de métal de transition ou de lanthanide,
dans un solvant,
pour conduire au composé de formule (X), sous forme racémique ou optiquement active : 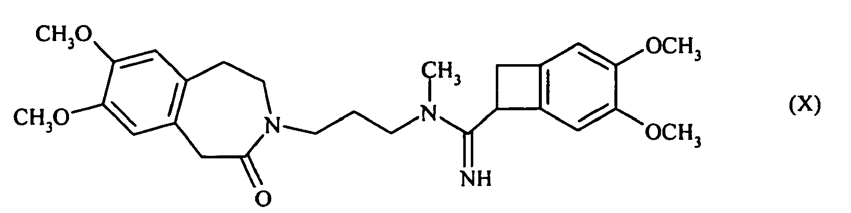 qui est transformé en composé de formule (VIII) par action d'un agent donneur d'hydrure.

2. Procédé de synthèse selon la revendication 1, **caractérisé en ce que** le composé de formule (II) est de configuration (*S*) et que le produit de la réaction du composé de formule (X) avec l'agent donneur d'hydrure est l'ivabradine de formule (I), cas particulier des composés de formule (VIII) : 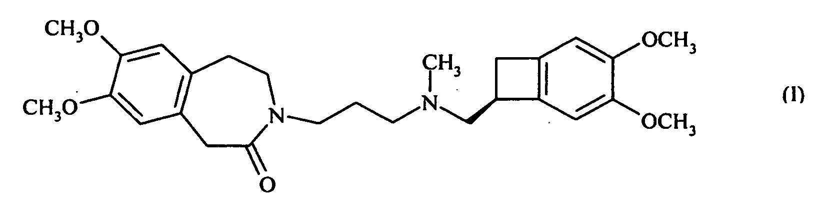 qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

3. Procédé de synthèse selon la revendication 1, **caractérisé en ce que** le composé de formule (II) est sous forme racémique et que la réaction du composé de formule (X) avec l'agent donneur d'hydrure est suivie d'une étape de résolution optique du composé de formule (VIII) obtenu sous forme racémique pour conduire à l'ivabradine de formule (I) : 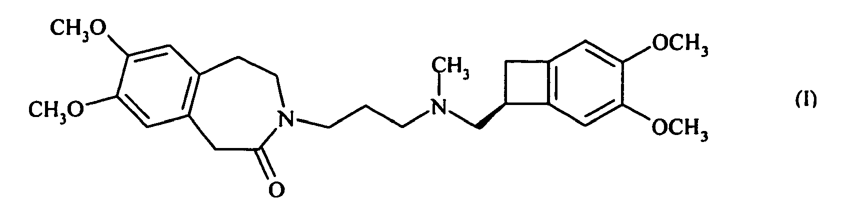 qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

4. Procédé de synthèse selon l'une quelconque des revendications 1 à 3, **caractérisé en ce que** le sel de métal de transition ou de lanthanide utilisé pour effectuer la réaction entre le composé de formule (II) et le composé de formule (IX) est choisi parmi le chlorure de cuivre(I), le bromure de cuivre(I), l'iodure de cuivre(I), le trifluorométhanesulfonate d'yttrium(III), le trifluorométhanesulfonate de lanthane(III), le trifluorométhanesulfonate de praséodyme(III), le trifluorométhanesulfonate de néodyme(III), le trifluorométhanesulfonate de samarium(III), le trifluorométhanesulfonate d'europium(III), le trifluorométhanesulfonate de gadolinium(III), le trifluorométhanesulfonate de terbium(III), le trifluorométhanesulfonate de dysprosium(III), le trifluorométhanesulfonate d'holmium(III), le trifluorométhanesulfonate d'erbium(III) et le trifluorométhanesulfonate de lutécium(III).

5. Procédé de synthèse selon l'une quelconque des revendications 1 à 4, **caractérisé en ce que** le solvant utilisé pour effectuer la réaction entre le composé de formule (II) et le composé de formule (IX) est choisi parmi les solvants alcooliques, le diméthylsulfoxyde, le *N,N*-diméthylformamide et la *N*-méthylpyrrolidone.

6. Procédé de synthèse selon l'une quelconque des revendications 1 à 5, **caractérisé en ce que** l'agent donneur d'hydrure utilisé pour effectuer la transformation du composé de formule (X) en composé de formule (VIII) est choisi parmi le tétraborohydrure de sodium, le cyanoborohydrure de sodium, le complexe borane-morpholine et le complexe borane-diméthylamine.

7. Composé de formule (X), sous forme racémique ou optiquement active:

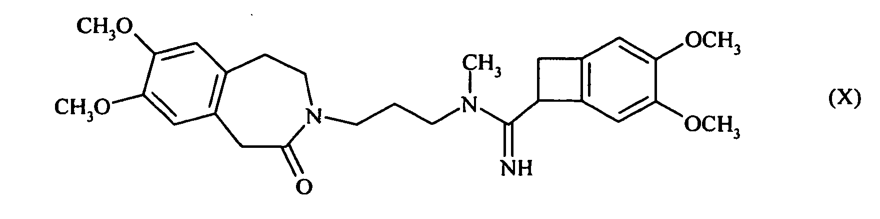

## Claims

1. Process for the synthesis of the compound of formula (VIII), in racemic or optically active form: 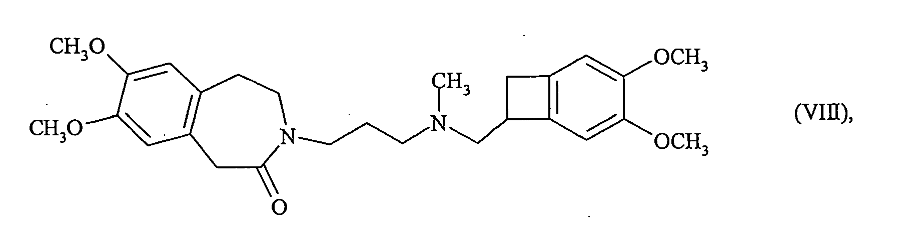 **characterised in that** the compound of formula (II), in racemic or optically active form: 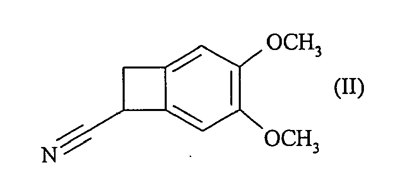 is reacted with the compound of formula (IX): 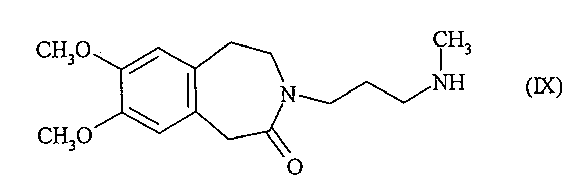 in the presence of a salt of a transition metal or of a lanthanide,
in a solvent,
to yield the compound of formula (X), in racemic or optically active form: 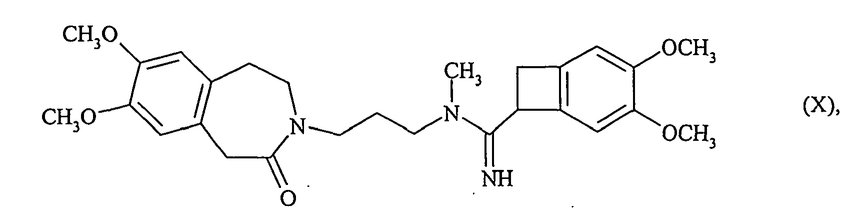 which is converted into the compound of formula (VIII) by the action of a hydride donor agent.

2. Synthesis process according to claim 1, **characterised in that** the compound of formula (II) is of configuration (*S*), and **in that** the product of the reaction of the compound of formula (X) with the hydride donor agent is ivabradine of formula (I), a particular case of the compounds of formula (VIII): 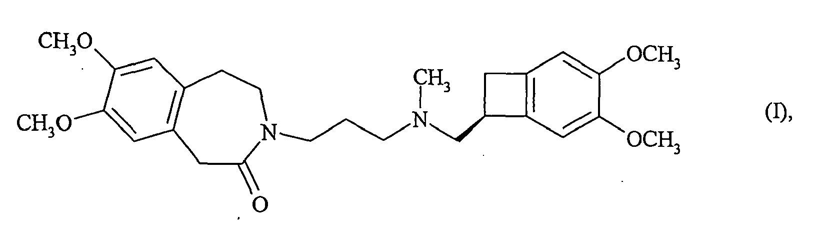 which may be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

3. Synthesis process according to claim 1, **characterised in that** the compound of formula (II) is in racemic form, and **in that** the reaction of the compound of formula (X) with the hydride donor agent is followed by a step of optical resolution of the compound of formula (VIII) obtained in racemic form, to yield ivabradine of formula (I): 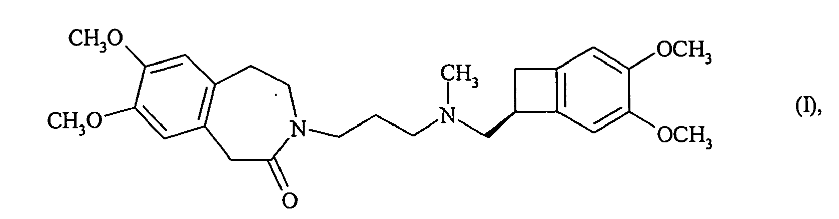 which may be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

4. Synthesis process according to any one of claims 1 to 3, **characterised in that** the salt of a transition metal or of a lanthanide used to carry out the reaction between the compound of formula (II) and the compound of formula (IX) is selected from copper(I) chloride, copper(I) bromide, copper(I) iodide, yttrium(III) trifluoromethanesulphonate, lanthanum(III) trifluoromethanesulphonate, praseodymium(III) trifluoromethanesulphonate, neodymium(III) trifluoromethanesulphonate, samarium(III) trifluoromethanesulphonate, europium(III) trifluoromethanesulphonate, gadolinium(III) trifluoromethanesulphonate, terbium(III) trifluoromethanesulphonate, dysprosium(III) trifluoromethanesulphonate, holmium(III) trifluoromethanesulphonate, erbium(III) trifluoromethanesulphonate and lutetium(III) trifluoromethanesulphonate.

5. Synthesis process according to any one of claims 1 to 4, **characterised in that** the solvent used to carry out the reaction between the compound of formula (II) and the compound of formula (IX) is selected from alcoholic solvents, dimethyl sulphoxide, *N,N*-dimethylformamide and *N*-methylpyrrolidone.

6. Synthesis process according to any one of claims 1 to 5, **characterised in that** the hydride donor agent used to carry out the conversion of the compound of formula (X) to the compound of formula (VIII) is selected from sodium tetraborohydride, sodium cyanoborohydride, the complex borane-morpholine and the complex borane-dimethylamine.

7. Compound of formula (X), in racemic or optically active form:

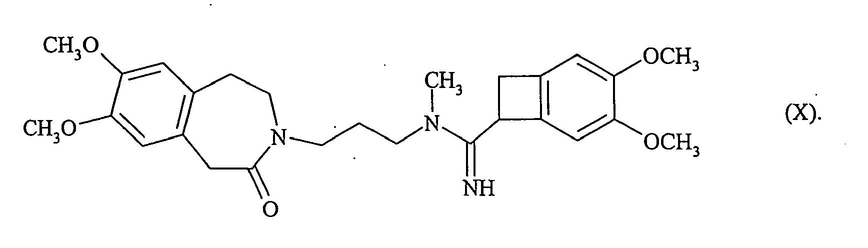

## Patentansprüche

1. Verfahren zur Synthese der Verbindung der Formel (VIII) in racemischer oder optisch aktiver Form: 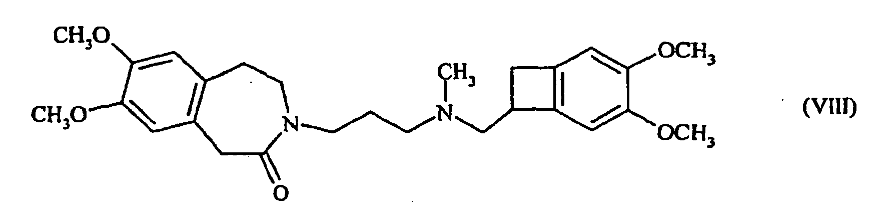 **dadurch gekennzeichnet, dass** man die Verbindung der Formel (II): 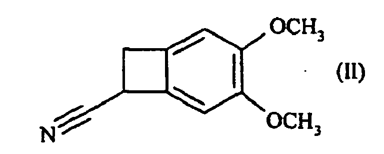 in racemischer oder optisch aktiver Form mit der Verbindung der Formel (IX): 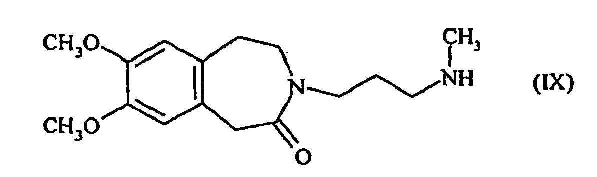 in Gegenwart eines Übergangsmetallsalzes oder eines Lanthanidensalzes
in einem Lösungsmittel umsetzt
zur Bildung der Verbindung der Formel (X) in racemischer oder optisch aktiver Form: 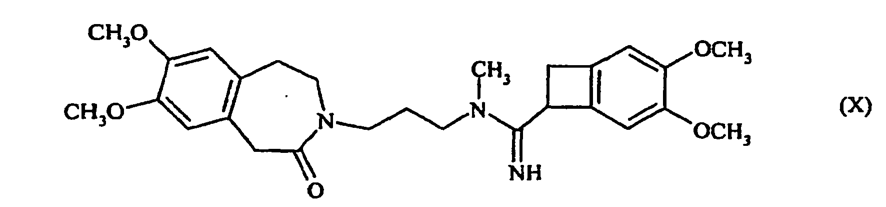 welche durch Einwirkung eines Hydriddonors in die Verbindung der Formel (VIII) umgewandelt wird.

2. Syntheseverfahren nach Anspruch 1, **dadurch gekennzeichnet, dass** die Verbindung der Formel (II) in der Konfiguration (*S*) vorliegt und das Produkt der Umsetzung der Verbindung der Formel (X) mit dem Hydriddonor Ivabradin der Formel (I) ist, einem Sonderfall der Verbindungen der Formel (VIII): 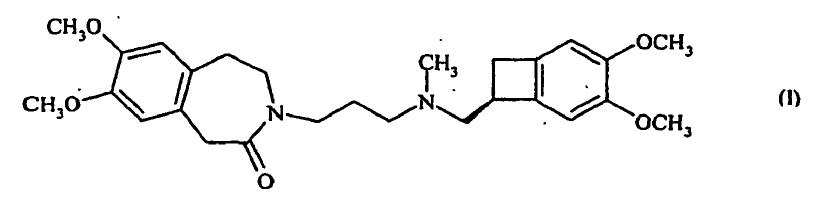 welche mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure in seine Additionssalze und in ihre Hydrate umgewandelt werden kann.

3. Syntheseverfahren nach Anspruch 1, **dadurch gekennzeichnet, dass** die Verbindung der Formel (II) in racemischer Form eingesetzt wird und die Umsetzung der Verbindung der Formel (X) mit dem Hydriddonor von einer Stufe der optischen Auftrennung der in racemischer Form erhaltenen Verbindung der Formel (VIII) gefolgt wird, zur Bildung von Ivabradin der Formel (I): 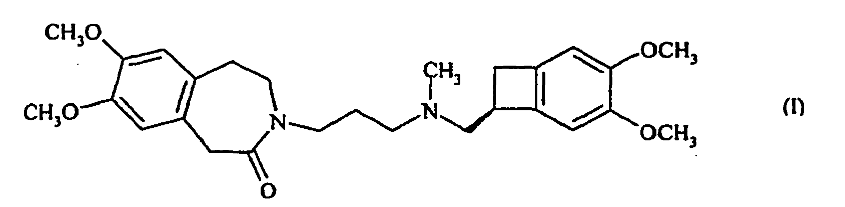 welches in seine Additionssalze mit einer pharmazeutisch annehmbaren Säure, ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure und deren Hydrate umgewandelt werden kann.

4. Syntheseverfahren nach einem der Ansprüche 1 bis 3, **dadurch gekennzeichnet, dass** das zur Durchführung der Reaktion zwischen der Verbindung der Formel (II) und der Verbindung der Formel (IX) eingesetzte Übergangsmetallsalz oder Lanthanidensalz ausgewählt ist aus Kupfer(I)-chlorid, Kupfer(I)-bromid, Kupfer(I)-iodid, Yttrium(III)-trifluormethansulfonat, Lanthan(III)-trifluormethansulfonat, Praseodym(III)-trifluormethansulfonat, Neodym(III)-trifluormethansulfonat, Samarium(III)-trifluormethansulfonat, Europium(III)-trifluormethansulfonat, Gadolinium(III)-trifluormethansulfonat, Terbium(III)-trifluormethansulfonat, Dysprosium(III)-trifluormethansulfonat, Holmium(III)-trifluormethansulfonat, Erbium(III)-trifluormethansulfonat und Lutetium(III)-trifluormethansulfonat.

5. Syntheseverfahren nach einem der Ansprüche 1 bis 4, **dadurch gekennzeichnet, dass** das für die Durchführung der Reaktion der Verbindung der Formel (II) mit der Verbindung der Formel (IX) verwendete Lösungsmittel aus alkoholischen Lösungsmitteln, Dimethylsulfoxid, *NN*-Dimethylformamid und *N*-Methylpyrrolidon ausgewählt wird.

6. Syntheseverfahren nach einem der Ansprüche 1 bis 5, **dadurch gekennzeichnet, dass** der für die Umwandlung der Verbindung der Formel (X) indie Verbindung der Formel (VIII) verwendete Hydriddonor aus Natriumtetraborhydrid, Natriumcyanborhydrid, dem Boran-Morpholin-Komplex und dem Boran-Dimethylamin-Komplex ausgewählt wird.

7. Verbindung der Formel (X) in racemischer oder optisch aktiver Form:
